# EUROPEAN PATENT APPLICATION

(11) **EP 4 792 825 A1**
(43) Date of publication of application: **19.08.2026**
(21) Application number: 25225361.2
(22) Date of filing: 19.12.2025
(51) Int. Cl.: A61K 38/16, A61K 47/64, A61L 27/22, A61L 27/54, A61P 21/00, A61K 38/08, A61P 21/04, C07K 14/00, C07K 19/00, A61K 38/10, C07K 7/06, C07K 7/08, C07K 7/18

(54) **IL 4 AND SDF 1 DERIVED PEPTIDES FOR ENHANCING SKELETAL MUSCLE REPAIR**

(30) Priority: 18.02.2025 EP 25461510
(71) Applicant: Uniwersytet Gdanski, 80-309 Gdansk (PL); Uniwersytet Warszawski, 00-927 Warszawa (PL)
(72) Inventor: Michalska, Zuzanna, 03-226 Warszawa (PL); Ostaszewska, Anna, 02-791 Warszawa (PL); Fularczyk, Martyna, 80-293 Gdansk (PL); Dzierzynska, Maria, 81-824 Sopot (PL); Bielak, Kacper, 05-092 Lomianki (PL); Morytz, Justyna, 03-252 Warszawa (PL); Archacka, Karolina, 05-816 Michalowice (PL); Brzoska-Wojtowicz, Edyta, 03-937 Warszawa (PL); Ciemerych-Litwinienko, Maria, 02-132 Warszawa (PL); Rodziewicz-Motowidlo, Sylwia, 83-010 Rotmanka (PL); Grabowska-Kowalik, Iwona, 02-384 Warszawa (PL); Zimowska-Wypych, Malgorzata, 01-627 Warszawa (PL); Janczyk-Ilach, Katarzyna, 03-757 Warszawa (PL); Streminska, Wladyslawa, 02-372 Warszawa (PL)
(74) Representative: Czarnik, Maciej

(57) **Abstract**

Short synthetic peptides derived separately from interleukin-4 (IL-4) and stromal cell-derived factor-1 (SDF-1/CXCL12), designed by structure-guided selection of receptor-binding regions (IL-4(5-15), IL-4(78-92), SDF-1(1-8), SDF-1(39-49)). When applied to mammalian cells at approximately 250 ng/mL, these peptides reproduce selected biological activities of the parent cytokines without causing off-target differentiation. IL-4-derived peptides enhance myoblast fusion and reduce adipogenic differentiation; SDF-1-derived peptides promote migration of fibroblasts, myoblasts, mesenchymal stromal cells and induced pluripotent stem cells. The peptides are non-toxic and suitable as components of cell culture media or biomaterial scaffolds for skeletal muscle regeneration.

## Description

The invention relates to biotechnology, tissue engineering and regenerative medicine. In particular, it concerns synthetic peptides derived from interleukin-4 (IL-4) and stromal cell-derived factor 1 (SDF-1/CXCL12), their use in activating corresponding receptors (IL-4Rα and CXCR4 ), and compositions, scaffolds and methods employing such peptides to promote migration, survival, fusion and differentiation of cells involved in skeletal muscle regeneration, including satellite cell-derived myogenic precursor cells (MPCs), myoblasts, fibroblasts, endothelial and stromal cells, as well as pluripotent stem cells.

### Background of the invention

Skeletal muscle regeneration and limitations. Skeletal muscle tissue is characterized by an outstanding ability to regenerate after damage resulting from exercise, accidental injuries, and diseases, such as dystrophies, or wasting accompanying cancer as well as aging. Under physiological conditions, skeletal muscle regeneration is secured by muscle specific unipotent stem cells, that is, satellite cells (SCs), which reside quiescently between the sarcolemma and the basal lamina surrounding myofiber [1]. Upon injury, these cells become activated, turn into rapidly proliferating muscle precursor cells (MPCs), then myoblasts, which eventually exit the cell cycle, convert into myocytes, and fuse to form multinucleated myotubes and finally mature myofibers. Simultaneously, myofibers become innervated, muscle regains proper vasculature, and the extracellular matrix (ECM) is reformed. Despite the fact that muscle regeneration is primarily dependent on the SCs differentiation, other cell types, such as fibroblasts, endothelial cells, or neurons are indispensable in the proper progression of this process [2]. Importantly, each time SCs become activated, they also undergo self-renewal, allowing them to retain their population and secure subsequent rounds of regeneration. Unfortunately, with aging or disease progression, the SCs pool may be exhausted, which could result in muscle failure to regenerate properly [3, 4]. Also, in the case of extensive muscle injuries, such as volumetric muscle loss (VML), the regenerative ability of the muscle is not sufficient for its proper reconstruction. For this reason, various strategies, such as cell-based ones, are considered to support skeletal muscle repair [5].

Cell-based therapies and their shortcomings. The first cells to be tested in muscle cell therapies were SCs or SCs-derived MPCs (SCs-MPCs). However, the usefulness of these cells was limited by the fact that after transplantation they fail to migrate and colonize regenerating skeletal muscle (e.g., [6]), undergo necrosis, apoptosis or anoikis (e.g., [6-8]), or are eliminated by the host immune system (e.g., [6]). Different experimental approaches to counteract these problems were tested, among them those involving various scaffolds and gels that allow one to improve cell survival and colonization of regenerating muscles (e.g., [9, 10]). Such biomaterials could be further developed by the application of additional factors. Next, cells other than SCs and SCs-MPCs were investigated as ones that could undergo myogenic differentiation and support skeletal muscle regeneration. Among them were those residing within the muscle: mesoangioblasts (e.g., [11]); pericytes [12]; muscle-resident interstitial cells that do not express Pax7 but synthesize the cell stress mediator PW1 [13]; or muscle side population cells [14]. However, none of these cells, including SCs, can be considered as easily available and expandable in vitro. Next, cells isolated from other tissues, such as bone marrow mesenchymal stromal cells/medicinal signaling cells (BMSCs) or stromal cells derived from adipose tissue were studied (e.g., [15, 16]). Unfortunately, also these do not have sufficient therapeutic potential due to their limited ability to undergo myogenic differentiation [17]. Among exogenous cells that could be used for stem cell-based therapies are also pluripotent stem cells (PSCs), such as induced pluripotent stem cells (iPSCs). PSCs can serve as a source of MPCs since multiple protocols have already been published showing myogenic differentiation of human iPSCs (hiPSCs) (e.g., [18, 19]).

Pro-regenerative signaling by IL-4 and SDF-1. IL-4 produced in regenerating muscle supports myoblast recruitment and multinucleated myotube formation and can modulate MPCs migration *in vitro* and *in vivo* [20,21]. SDF-1 (CXCL12) is a master regulator of stem cell migration/homing, expressed mostly in bone marrow, but also in skeletal muscle, which function is to enhance myoblast fusion by up-regulating the expression of adhesion protein CD9 during regeneration [22,23,27-29]. SDF-1 also protects stromal cells from apoptosis and promotes wound healing [30]. Prior work demonstrates IL-4 and SDF-1 can improve the ability of adipose-derived stromal cells to support myogenic regeneration and influence myogenic differentiation of embryonic stem cells [24-26,36].

Need for targeted, safer modulators within biomaterials. Full-length cytokines and growth factors can produce adverse effects including inflammation or off-target signaling [31,32]. Short synthetic peptides that mimic receptor-interacting regions can recapitulate desired signaling with improved safety, tunable stability, and ability to be used as chemical functionalization agents for controlled release from biomaterial scaffolds [33-35]. However, there are no peptide-based IL-4 or SDF-1 fragments specifically engineered for metalloproteinase-triggered release within muscle lesions and validated for pro-regenerative actions across relevant human cell types.

In the patent documents present in the state of the art, there are disclosed inventions based on IL-4-derived peptides. For example, the European patent application EP3530320 A1 discloses a new type of compound made up of specific sequences of amino acids that are derived from proteins (human or animal IL-4 or IL-13) involved in immune responses, which could help in nerve regeneration. These amino acid sequences are designed to promote the growth of nerve fibers, potentially aiding recovery from injuries or diseases affecting the nervous system.

US2011300148 AA relates to IL-4 protein fragments capable of binding to the IL-4 receptors and inhibiting macrophage activation, and thereby preventing the onset of inflammatory response. The invention further relates to use of said peptides for the production of a medicament for the treatment of different pathological conditions, wherein IL-4 plays a prominent role. No applications to skeletal muscle regenerationwere disclosed.

Peptide fragments derived from interleukin-4 (IL-4) for the treatment of diabetic nephropathy are disclosed in US2025064887 AA. In this approach specific parts of a well-known immune system protein are used to target and treat kidney damage, rather than using whole proteins or traditional medications. No uses relevant to skeletal muscle regeneration were mentioned in the document.

US patent no. US6875738B1 discloses a variety of therapeutic uses for CXCR4 antagonists derived from SDF-1 amino acid sequence. The CXCR4 antagonists may be used as therapeutics, or to manufacture a medicament, for reducing interferon gamma production by T-cells, treatment of an autoimmune disease, treatment of multiple sclerosis, treatment of cancer, inhibition of angiogenesis.

The aim of the invention is to provide biologically active compounds, suitable for treatment, that: (i) enhance migration of myogenic and supportive cells, (ii) preserve cytoskeletal organization, (iii) avoid undesirable lineage differentiation (e.g., adipo-/osteo-/chondrogenesis), and (iv) promote myoblast fusion, while enabling localized, enzyme-responsive release compatible with injured skeletal muscle microenvironments.

### Summary of the invention

The essence of the invention is a synthetic peptide selected from: (a) LITLQEIIKTLN (SEQ ID NO: 1, IL-4-X), (b) LQLIRFLKRLDRNLWG (SEQ ID NO: 2, IL-4-Y), (c) LKPVSLSYR (SEQ ID NO: 3, SDF-1-X), (d) LVARLKNNNRQV (SEQ ID NO: 4, SDF-1-Y), wherein the said peptide comprises an N-terminal Leu residue enabling MMP-sensitive conjugation.

In another aspect, the invention relates to a peptide-based composition for modulating cellular processes involved in skeletal muscle regeneration, wherein the composition comprises at least one synthetic peptide selected from SEQ ID NO: 1-4, wherein each peptide is covalently tethered to a biomaterial scaffold through an MMP-cleavable linker selected from VLG↓L, PLG↓L, PAG↓L, PLN↓L, PAN↓L or VAL↓L.

Preferably, the peptide-based composition of the invention comprises SEQ ID NO: 1 or SEQ ID NO: 2, and said peptide binds to and activates the IL-4 receptor α to enhance fusion of skeletal myoblasts and decrease adipogenic differentiation of fibroblasts.

Preferably, the peptide-based composition of the invention comprises SEQ ID NO: 3 or SEQ ID NO: 4, and said peptide binds to and activates CXCR4 receptors to enhance migration of fibroblasts, skeletal muscle precursor cells, or mesenchymal stromal cells.

Preferably, the biomaterial scaffold of the peptide-based composition of the invention is chosen from the list: PEG-based hydrogels, chitosan sponges, and polymeric, synthetic hydrogels. Preferably, peptide is present at a concentration from 50 ng/mL to 1000 ng/mL, most preferably about 250 ng/mL

In another aspect, the invention relates to the synthetic peptide of the invention or the peptide-based composition of the inventions for use as a medicament.

The synthetic peptide according to claim 1 or the peptide-based composition according to any of claims 2 to 6 for use in enhancing skeletal muscle repair and promoting regeneration of skeletal muscle by modulating human dermal fibroblasts, skeletal myoblasts, bone-marrow mesenchymal stromal cells induced pluripotent stem cells.

A use of the synthetic peptide of the invention or the peptide-based composition of the invention for promoting regeneration of injured skeletal muscle in a mammal. Preferably, when said composition is administered together with induced pluripotent stem cells, or myogenic cells (myoblasts) to further enhance skeletal muscle repair.

Advantages of claimed invention is that it provides peptides to be used in targeted and modular ways: choice of one peptide family (IL-4-derived or SDF-1-derived) according to the therapeutic goal. Provided peptides are safe in use by the absence of ectopic osteo/chondrogenesis, reduced adipogenesis with IL-4 peptides; preserved cytoskeletal integrity. There are also advantages in the manufacturing process due to the short sequences of claimed peptides, straightforward SPPS *(Solid Phase Peptide Synthesis)* synthesis, amenable to ionexchange counterion optimization and analytical control (HPLC, LC-MS). Peptides of the invention can be validated mechanistically: CD (Figure 1), MST for IL-4 (Figure 2), receptor expression (Figure 5), signaling (Figure 6), functional cell assays (Figures 7-10).

The subject matter of the invention is presented in examples that do not limit its scope and in a drawing in which:

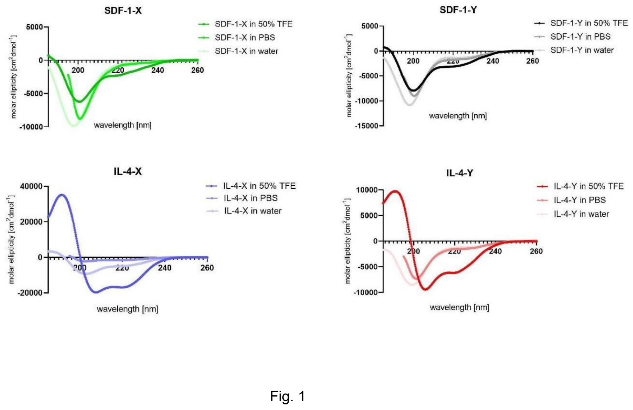

- Fig. 1.: illustrates CD spectra of IL-4 (SEQ ID NO: 1; SEQ ID NO: 2) and SDF-1-derived (SEQ ID NO: 3; SEQ ID NO: 4) peptides in water, in 50% H₂O/TFE or phosphate buffer.

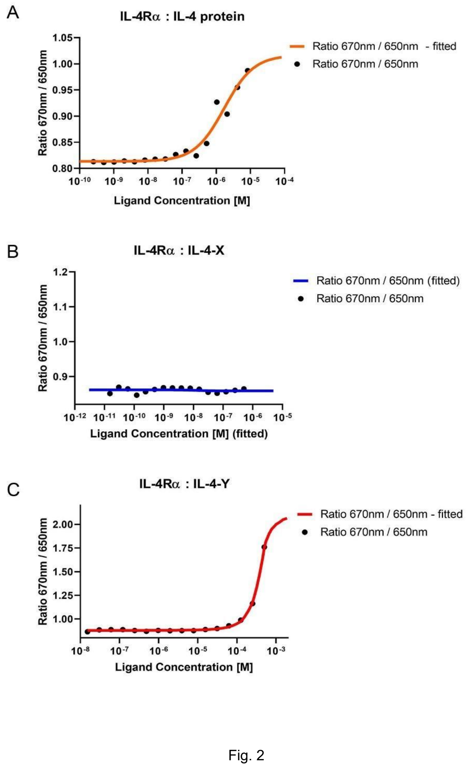

- Fig. 2.: illustrates dose response curves for IL-4Rα Receptor interaction with IL-4 protein (A), SEQ ID NO: 1 (IL-4-X) peptide (B) and SEQ ID NO: 2 (IL-4-Y) peptide (C). Calculated K_{D} for IL-4 protein is 1.7 µM and SEQ ID NO: 2 (IL-4-Y) is 349 µM.

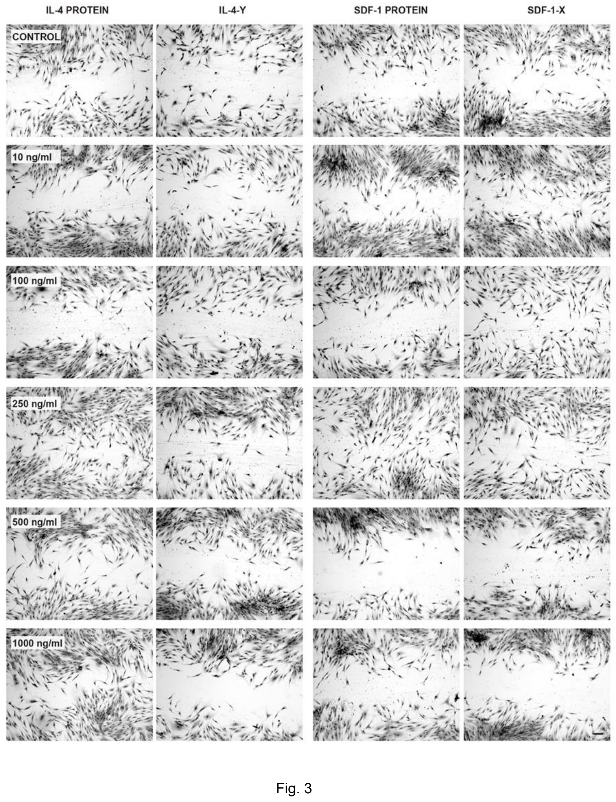

- Fig. 3.: illustrates results for several concentrations, i.e. 0 ng/ML, 10 ng/ML, 100 ng/ML, 250 ng/mML, 500 ng/ML, and 1000 ng/ML, of either human IL-4 and SDF-1 proteins, or SEQ ID NO: 2 (IL-4-Y) and SEQ ID NO: 3 (SDF-1-X) peptides were used to evaluate their impact on hDF migration. Representative pictures of scratch wound healing assay after 12 hours in culture were presented. Scale bar = 100 µm

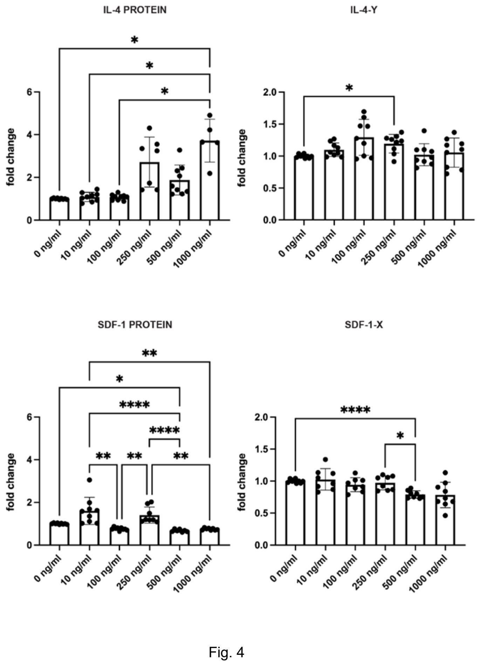

- Fig. 4.: illustrates quantitative evaluation of scratch wound healing assay from Fig. 3. The data is shown as a fold change of mean scratch wound area in control (0 ng/ML) and scratch wound area in cells maintained in media supplemented with either proteins (IL-4 or SDF-1) or peptides (SEQ ID NO: 4 (IL-4-Y) or SEQ ID NO: 3 (SDF-1-X)). The data is shown as the mean value of at least five independent replicates. * p<0.05, ** p<0.01, *** p<0.001, **** p<0.0001

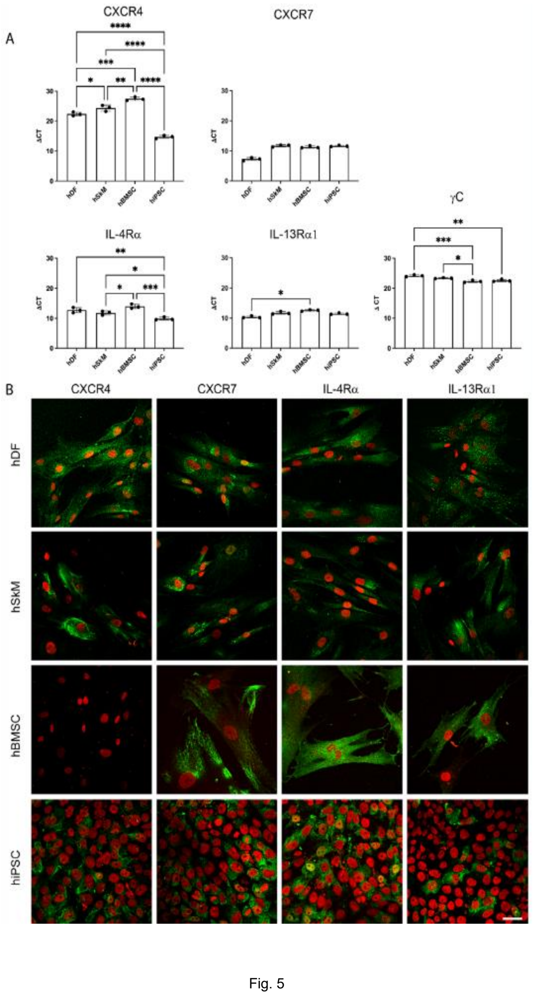

- Fig. 5.: illustrates results of analysis of IL-4 and SDF-1 receptor expression. A. RT-qPCR analysis of the expression of mRNA transcripts encoding IL-4 (*IL*-4R*α* and *IL-13Rα1*) and SDF-1 (*CXCR4* and *CXCR7)* receptors in different cell populations, i.e., hDFs, hSkMs, hBMSCs, hiPSCs. The results were shown as delta CT; B. Immunolocalization of IL-4 (IL-4Rα and IL-13Rα1) and SDF-1 (CXCR4 and CXCR7) receptors in tested cell populations. The receptors were visualized with green and cell nuclei in red. Scale bar: 50 µm.

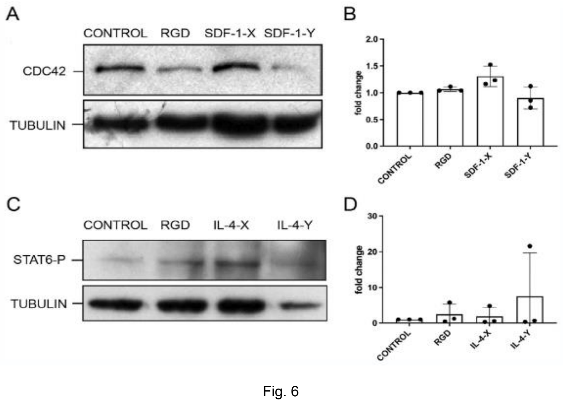

- Fig. 6.: illustrates results of western blot analysis of CDC42 and phospho-STAT-6 in hDFs cultured either in the medium lacking peptides (control) or SEQ ID NO: 5 (RGD), SEQ ID NO: 1 (IL-4-X), SEQ ID NO: 2 (IL-4-Y), SEQ ID NO: 3 (SDF-1-X), or SEQ ID NO: 4 (SDF-1-Y) supplemented medium. A. representative CDC42 immunoblot; C. representative phospho-STAT-6 immunoblot; Tubulin levels were used as an internal reference. The graphs B and D present results of densitometric analysis of three independent Western blots, shown as a fold change compared to cells cultured in control medium.

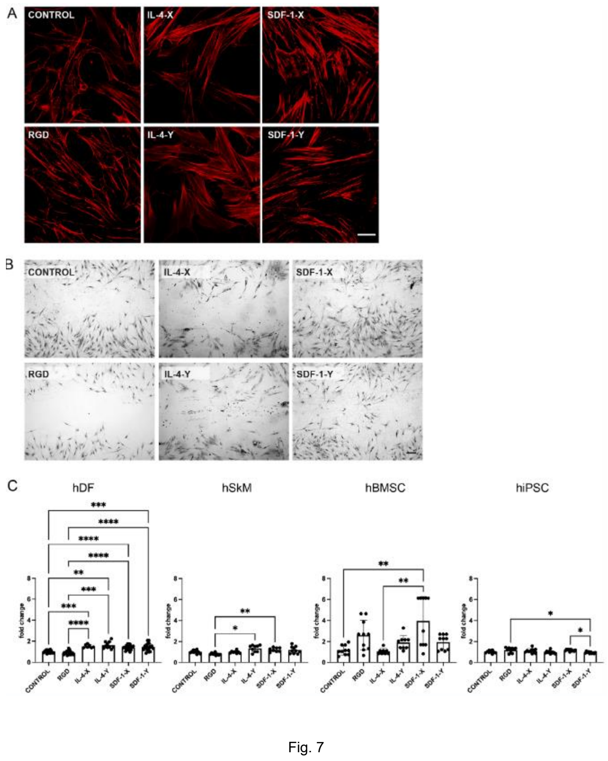

- Fig. 7.: illustrates hDFs morphology and migration of hDFs, hSkMs, hBMSCs, and hiPSCs cultured either in the medium lacking peptides (control) or SEQ ID NO: 5 (RGD), SEQ ID NO: 1 (IL-4-X), SEQ ID NO: 2 (IL-4-Y), SEQ ID NO: 3 (SDF-1-X), or SEQ ID NO: 4 (SDF-1-Y) supplemented medium. A. Immunolocalization of actin (red) in hDFs after 24h of peptide treatment. Actin cytoskeleton shown in red. Scale bar 50 µm; B. Images of hDFs 24h after the scratch test was performed. Cells were stained with Giemsa and May-Grünwald; C. Migration rate of cells cultured in the presence of peptides and calculated as fold change of the data obtained for cells cultured in control medium. * p<0.05, ** p<0.01, *** p<0.001, **** p<0.0001

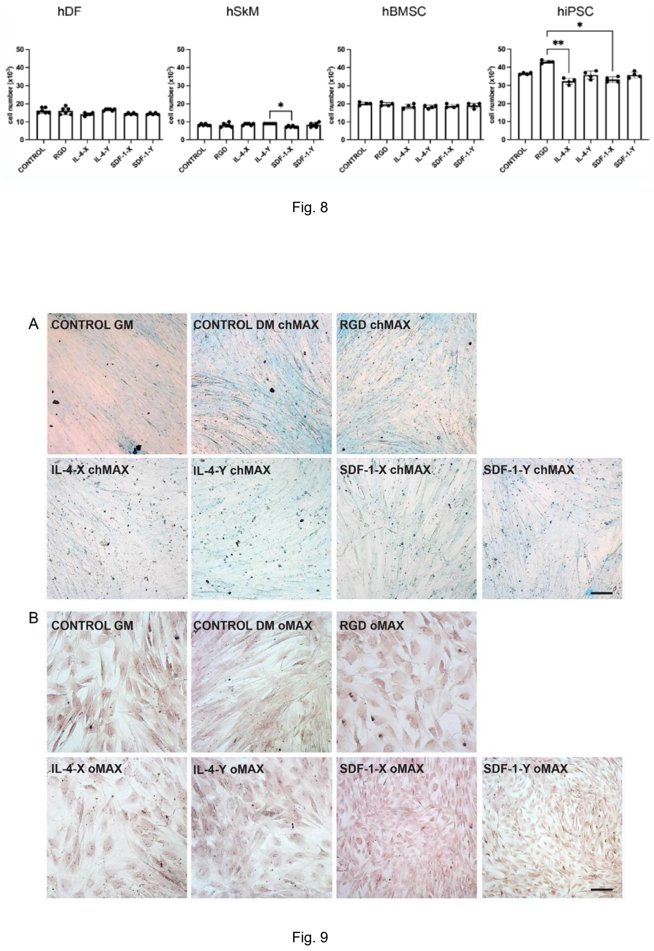

- Fig. 8.: illustrates proliferation of hDFs, hSkMs, hBMSCs, and hiPSCs cultured either in the medium lacking peptides (control) or SEQ ID NO: 5 (RGD), SEQ ID NO: 1 (IL-4-X), SEQ ID NO: 2 (IL-4-Y), SEQ ID NO: 3 (SDF-1-X), or SEQ ID NO: 4 (SDF-1-Y) supplemented medium. Cells were seeded in the density of 5000 cells/plate and cell number was analyzed after three days in culture. Samples cultured in the presence of peptides were compared either to cell cultures in control or SEQ ID NO: 5 (RGD) containing medium. * p<0.05, ** p<0.01
- Fig. 9.: illustrates chondrogenic and osteogenic differentiation of hDFs cultured either in the differentiation medium lacking peptides (control) or SEQ ID NO: 5 (RGD), SEQ ID NO: 1 (IL-4-X), SEQ ID NO: 2 (IL-4-Y), SEQ ID NO: 3 (SDF-1-X), or SEQ ID NO: 4 (SDF-1-Y) supplemented medium. A. hDF culture stained with alcian blue after inducing chondrogenic differentiation; B. hDF culture stained with alizarin red S after inducing osteogenic differentiation. Scale bar 100 µm

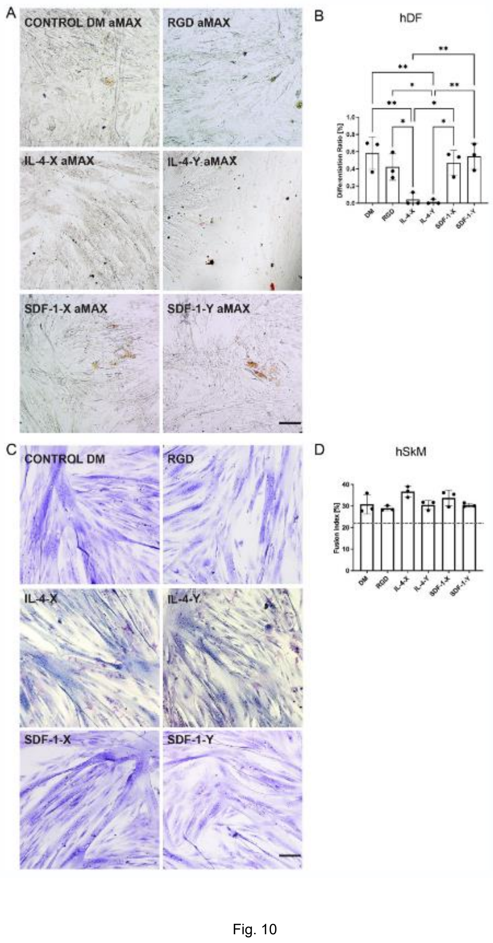

- Fig. 10.: illustrates adipogenic differentiation of hDFs and myogenic differentiation of hSkM cultured either in the media lacking peptides (control) SEQ ID NO: 5 (RGD), SEQ ID NO: 1 (IL-4-X), SEQ ID NO: 2 (IL-4-Y), SEQ ID NO: 3 (SDF-1-X), or SEQ ID NO: 4 (SDF-1-Y) supplemented media. A. Cells stained with oil red O; Scale bar 100 µm; B. Quantification of cell differentiated area referred to total cell area, described as differentiation ratio, and compared either to cells cultures in control or SEQ ID NO: 5 (RGD) containing medium. * p<0.05, ** p<0.01, *** p<0.001; C. Myotubes stained with Giemsa-May Grünwald. Scale bar: 100 µm; C. Fusion index calculated as a proportion of nuclei inside myotubes divided by total number of nuclei in the view field, compared either to cells cultures in control or SEQ ID NO: 5 (RGD) containing medium. * p<0.05, ** p<0.01, *** p<0.001.

The invention provides a family of synthetic bioactive peptides (SEQ ID NO: 1 (IL-4-X), SEQ ID NO: 2 (IL-4-Y), SEQ ID NO: 3 (SDF-1-X), and SEQ ID NO: 4 (SDF-1-Y) - see Table 1) derived separately from interleukin-4 (IL-4) and stromal cell-derived factor-1 (SDF-1/CXCL12) that are engineered for covalent attachment to biomaterial scaffolds including PEG-based hydrogels, chitosan sponges, and polymeric, synthetic hydrogels and for protease-responsive (MMPs) release within the milieu of injured skeletal muscle via cleavable linkers (e.g., VLG↓L, PLG↓L, PAG↓L, PLN↓L, PAN↓L, VAL↓L). The peptides are short, chemically tractable, and recapitulate receptor-engaging regions of the native ligands while avoiding adverse effects potentially triggered by whole cytokines.

**Table 1. Amino-acid sequences of peptides and designed MMP-cleavable motifs.**

| No | Peptide name | Seq. No | Amino acid sequence | Seq. length |
|---|---|---|---|---|
| 1. | IL-4-X | SEQ ID NO: 1 | NH₂-↓LITLQEIIKTLN-NH₂ | 12 |
| 2. | IL-4-Y | SEQ ID NO: 2 | NH₂-↓LQLIRFLKRLDRNLWG-NH₂ | 16 |
| 3. | SDF-1-X | SEQ ID NO: 3 | NH₂-↓LKPVSLSYR-NH₂ | 9 |
| 4. | SDF-1-Y | SEQ ID NO: 4 | NH₂-↓VARLKNNNRQV-NH₂ | 12 |
| 5. | RGD | SEQ ID NO: 5 | NH₂-↓LRGD-NH₂ | 4 |

Arrow "↓" indicates the enzymatic cleavage position in the designed MMP-sensitive junction (Section 3.3), not used in current in-vitro assays.

**Table 2. The amino acid sequences of synthesized peptides and their calculated (Ma - average mass and Mm - monoisotopic mass) and experimental MS (Mex) masses.**

| No | Peptide name | Amino acid sequence | Ma | Mm | Mex |
|---|---|---|---|---|---|
| 1. | IL-4-X | NH₂-LITLQEIIKTLN-NH₂ | 1397.72 | 1396.87 | 1396.62 |
| 2. | IL-4-Y | NH₂-LQLIRFLKRLDRNLWG-NH₂ | 2040.49 | 2039.22 | 2039.89 |
| 3. | SDF-1-X | NH₂-LKPVSLSYR-NH₂ | 1061.29 | 1060.64 | 1060.57 |
| 4. | SDF-1-Y | NH₂-LVARLKNNNRQV-NH₂ | 1423.68 | 1422.85 | 1422.76 |
| 5. | RGD | NH₂-LRGD-NH₂ | 458.52 | 458.26 | 458.26 |

First IL-4- or SDF-1-derived peptides purpose-built for skeletal muscle regeneration and MMP-triggered release. The invention teaches distinct peptide fragments of either IL-4 or SDF-1 (not co-administered) that can be tethered to scaffolds via MMP-cleavable linkers to achieve onsite, enzyme-regulated delivery in regenerating muscle (Section 3.3; Figure 1C,F; Table 1). Selection of SEQ ID NO: 1 (IL-4-X) (A-helix) and SEQ ID NO: 2 (IL-4-Y) (C-helix) was guided by their participation in the ligand-receptor interface within the IL-4/IL-4Rα complex; likewise, SEQ ID NO: 3 (SDF-1-X) (N-terminus 1-8) and SEQ ID NO: 4 SDF-1-Y (β-hairpin 39-49) were chosen for their interface-forming residues in the SDF-1/CXCR4 complex.

Biophysical confirmation of structure-forming propensity. Circular dichroism (Figure 1) shows SEQ ID NO: 1 (IL-4-X) and SEQ ID NO: 2 (IL-4-Y) gain α-helical content in 50% H₂O/TFE solution (water/trifluoroethanol), whereas SDF-1 peptides are largely disordered, consistently with the native SDF-1 N-terminus behavior during CXCR4 engagement.

Direct binding readout for IL-4/IL-4Rα by MST. MST (microscale thermophoresis) established K_{D} = 1.7 µM for IL-4 and K_{D} = 349 µM for SEQ ID NO: 2 (IL-4-Y), while SEQ ID NO: 1 (IL-4-X) showed no MST-detectable binding (Fig. 2). MST of the interactions between SEQ ID NO: 3 (SDF-1-X) or SEQ ID NO: 4 (SDF-1-Y) and CXCR4 was not feasible due to the fact that the CXCR4 receptor is a membrane protein. Instead, based on molecular docking analysis, SEQ ID NO: 3 (SDF-1-X) was indicated as the preferred CXCR4-targeted fragment.

Cell-type-specific functional benefits. Across human hDF, hSkM, hBMSC, hiPSC models, peptides did not perturb actin architecture (Figure 7A), did not induce chondrogenesis/osteogenesis (Figure 9A-B), reduced adipogenesis for IL-4 peptides (Figure 10A-B), enhanced migration in a cell- and peptide-specific manner (Figure 7B-C), and improved myoblast fusion for SEQ ID NO: 1 (IL-4-X) (Figure 10C-D). Peptide concentration used for media preparation *in vitro:* 250 ng/mL (Methods 2.5-2.9, Figures 3-4).

Importantly: the invention does not require or claim simultaneous administration of IL-4-derived and SDF-1-derived peptides. Each peptide family is an independent tool, to be selected according to the desired cellular outcome (e.g., SEQ ID NO: 1 (IL-4-X) for myoblast fusion; SEQ ID NO: 3 (SDF-1-X) for migration), and delivered individually via MMP-cleavable, scaffold-tethered constructs. Despite no binding being detected by MST for SEQ ID NO: 1 (IL-4-X) and IL-4Rα, the peptide's functional effect may be mediated via engagement of a co-receptor complex or through allosteric modulation that eludes detection in the MST setup.

### Representative outcomes

### IL-4-derived peptides (SEQ ID NO: 1 (IL-4-X), SEQ ID NO: 2 (IL-4-Y))

| | |
|---|---|
| Target: | IL-4Rα ( IL-13Rα1 or yC); expression confirmed at mRNA and protein levels in hDF, hSkM, hBMSC, hiPSC (Figure 5). |
| Signaling readout: | Increased STAT6-P tendency in treated hDFs (Figure 6C-D). |
| Migration: | hDF ↑ for SEQ ID NO: 1 (IL-4-X) and SEQ ID NO: 2 (IL-4-Y), compared to control and RGD; hSkM ↑ for SEQ ID NO: 2 (IL-4-Y), compared to SEQ ID NO: 5 (RGD); hBMSC - no difference; hiPSC - no difference (Figure 7C). |
| Proliferation: | Generally neutral; hiPSC↓ with SEQ ID NO: 1 (IL-4-X), compared to SEQ ID NO: 5 (RGD) (Figure 8). |
| Differentiation: | Adipogenesis↓ (hDF) with SEQ ID NO: 1 (IL-4-X)/SEQ ID NO: 2 (IL-4-Y) (Figure 10A-B); myoblast fusion↑ with SEQ ID NO: 1 (IL-4-X) (Figure 10C-D). |
| *SDF-1-derived peptides (SEQ ID NO: 3 (SDF-1-X), SEQ ID NO: 4 (SDF-1-Y))* | |
| Target: | CXCR4/CXCR7 (ACKR3); expression confirmed at mRNA and protein level in hDF, hSkm, hBMSC, except for CXCR4 protein detection in hBMSC (Figure 5). |
| Signaling readout: | CDC42 activation↑ with SEQ ID NO: 3 (SDF-1-X), non significant (Figure 6A-B). |
| Migration: | robust hDF↑ (SEQ ID NO: 3 (SDF-1-X), SEQ ID NO: 4 (SDF-1-Y), compared to control and SEQ ID NO: 5 (RGD)); hSkM↑ (SEQ ID NO: 3 (SDF-1-X), compared to SEQ ID NO: 5 (RGD)); hBMSC↑ (SEQ ID NO: 3 (SDF-1-X), compared to control); hiPSC↓ vs SEQ ID NO: 5 (RGD) and SEQ ID NO: 3 (SDF-1-X) for SEQ ID NO: 4 (SDF-1-Y) (Figure 7C). |
| Proliferation: | Mostly neutral; hSkM↓ with SEQ ID NO: 3 (SDF-1-X) vs SEQ ID NO: 2 (IL-4-Y); hiPSC↓ with SEQ ID NO: 3 (SDF-1-X) vs SEQ ID NO: 5 (RGD) (Figure 8). |
| Differentiation: | No chondro/osteo induction (Figure 9); adipogenesis unaffected or minimal; no negative effect on myogenesis. |

### Compositions and delivery formats

Scaffold-tethered peptides with MMP-cleavable junctions (e.g., sc-PLG↓L-pf and the motifs listed in Section 3.3) enabling localized release, where "sc" denotes the biomaterial scaffold domain and "pf" denotes the bioactive peptide fragment, with cleavage occurring at the indicated ↓ site to liberate the peptide in the injured muscle milieu. Media supplements for ex vivo cell preparation at 250 ng/mL as per Methods.

### Detailed description of the invention

This invention concerns short peptides derived separately from IL-4 or SDF-1 that recapitulate receptor-engaging regions and can be engineered (by sequence design) for future covalent attachment to biomaterials with metalloproteinase-cleavable release. In the present work all biological assays used free peptides in solution (250 ng/mL). The invention uses biophysical assays, and *in vitro* cell tests on human dermal fibroblasts (hDFs), human skeletal myoblasts (hSkMs), human bone-marrow mesenchymal stromal/medicinal signaling cells (hBMSCs), and human induced pluripotent stem cells (hiPSCs). Peptides synthesized are listed in Table 1:

### IL-4-receptor-targeting peptides

Residue-specific contribution of IL-4 to its receptor IL-4Rα was established by analysis of the contact interactions between the two proteins based on the crystal complex (PDB code: 3BPL) . Based on the results, 25 amino acids located in the A and C α-helices of IL-4 were involved in interactions with IL-4Rα. Specifically, fragments 5-15 (from helix A) and 78-92 (from helix C) of IL-4 were found to exhibit the highest number of contacts at the interface with IL-4Rα in the complex. Next, the two fragments of IL-4 I⁵TLQEIIKTLN¹⁵-IL-4(5-15), described as SEQ ID NO: 1 (IL-4-X) and Q⁷⁸LIRFLKRLDRNLWG⁹²-IL-4(78-92), described as SEQ ID NO: 2 (IL-4-Y), of IL-4 were chosen for further studies as potential activators of IL-4Rα receptor.

### SDF-1 receptor targeting peptides

Molecular docking revealed that the arrangement and shape of Lys1 to Leu5 residues at the N-terminus of SDF-1 are crucial for interaction with the CXCR4 receptor. NMR experiments have shown that SDF-1 possesses an unordered N-terminus (residues 1-8), which contains key residues for CXCR4 activation, particularly Lys1 and Pro2. Additionally, electrostatic attraction may facilitate interaction of Arg8, Arg12, Arg41, and Arg47 of SDF-1 with residues Glu2, Asp10, Glu14, Glu15, Asp20, and Asp22 at the N-terminus of CXCR4. Further, it should be noted that three receptor residues Asp97, Asp187, and Glu288 are crucial for chemokine binding; specifically, they are likely to form interactions with the first two residues of SDF-1, Lys1 and Pro2. Huang et al. showed that the electrostatic potential distribution of an energy-minimum conformation indicates that two negative electrostatic potential sites on CXCR4 would cooperatively interact with the positively charged groups of the SDF-1 ligand. Furthermore, inspection of the experimentally determined structure of the SDF-1/CXCR4 complex (PDB: 8U4O) indicates that the N-terminal segment K1-P2 and the β-hairpin segment V39-V49 of SDF-1 lie at the ligand-receptor interface and establish direct contacts with CXCR4 (see Figure 1). Based on these data and the complex structure, we designed two peptides K¹PVSLSYR⁸-SDF-1(1-8), described as SEQ ID NO: 3 (SDF-1-X) and V³⁹ARLKNNNRQV⁴⁹ SDF-1(39-49), described as SEQ ID NO: 4 (SDF-1-Y) with potential receptor-activating properties. Both peptides correspond to interface-contacting sequences in the SDF-1/CXCR4 complex and contain essential amino acid residues found in the SDF-1 protein; they carry a net positive charge, enabling interaction with the negatively charged surface or cavities of the CXCR4 receptor. Furthermore, theoretical and experimental studies for this complex or its mutants and peptide fragments are known in the literature, which clearly demonstrate that the N-terminus of the SDF-1 protein, comprising residues 1-8, is crucial for receptor activation. In addition, research by Luo found that the highly positive β-hairpin region of SDF-1 located within residues 39-47 (as in the binding of SEQ ID NO: 4 (SDF-1-Y)) is involved in CXCR4, possibly through electrostatic interaction.

### Design of peptides derived from IL-4 and SDF-1 recognized by metalloproteinases

Fragments of IL-4 and SDF-1 were designed to enable covalent attachment to the scaffold and gradual release via metalloproteinase-specific sequences. We designed covalent bonds to ensure future release of the peptides from the scaffold into the surrounding environment. In our reasoning the release mechanism is facilitated by incorporating specific amino acid sequences, namely sc-VLG↓L-pf (sc - scaffold; pf - protein fragment), sc-PAG↓L-pf, sc-PLN↓L-pf, sc-PLG↓L-pf, sc- PAN↓L-pf, or sc-VAL↓L-pf, within the peptide sequence. These sequences are sensitive to various metalloproteinases (MMPs), with the symbol "↓" indicating the enzymatic cleavage site [61-63]. Consequently, peptides containing the active peptide sequence of IL-4 or SDF-1 protein fragments, with the cleavable Leu (L) amino acid (located on the right side of the arrow), such as leucine (L), at the N- terminus of SDF-1(1-8), are expected to be released into the environment. Therefore, peptides that had been previously selected from IL-4 or SDF-1 proteins were synthesized (Table 1), with additional Leu as the first amino acid in the sequence, and then subjected to biological studies. As a control for the above-mentioned peptides, we used SEQ ID NO: 5 (RGD) (Arginyl-glycyl-aspartic acid) adhesion motif peptide, which does not bind neither to IL-4 nor SDF-1 receptors.

### Conformational studies of IL-4 or SDF-1-derived peptides using the Circular Dichroism method

Conformational studies of peptides aim to understand the structures they adopt in solution and to understand whether these structures resemble those found in the originating proteins on their amino acid sequences. These investigations are carried out in diverse solvents to mimic cellular conditions (such as water, PBS, etc.) or protein environments (TFE, DMSO, etc.). Therefore, we conducted similar examinations for peptides derived from IL-4 and SDF-1. Circular dichroism (CD) spectra of all studied peptides, measured in water and PBS, exhibit negative mean residue molar ellipticity values at a wavelength of approximately 199 nm (Figure 1), indicative of a disordered structure. Notably, the SEQ ID NO: 1 (IL-4-X) peptide stands out as it forms elements of an α-helical structure in water, evidenced by a maximum at around 190 nm and two minima at wavelengths of 204 nm and 222 nm. In a 50% H₂O/TFE solution, both SEQ ID NO: 1 (IL-4-X) and SEQ ID NO: 2 (IL-4-Y) peptides tend to adopt a helical structure, with the SEQ ID NO: 1 (IL-4-X) peptide displaying a higher helical structure compared to the SEQ ID NO: 2 (IL-4-Y) peptide. This is evident from the presence of a maximum at about 190 nm and two minima of almost equal depth at wavelengths of approximately 204 nm and 222 nm, characteristic of an α-helix structure. In contrast, the SEQ ID NO: 2 (IL-4-Y) peptide exhibits unstructured elements, indicated by a much deeper minimum at 204 nm compared to 222 nm. Both SDF-1 derived peptides in 50% H₂O/TFE solution form only elements of the helical structure, as shown by the appearance of a flat minimum at approximately 222 nm in their CD spectra. Circular dichroism analysis was performed to evaluate the structural similarity between IL-4 and SDF-1 derived peptides and their respective full-length proteins. Our findings indicate that the studied peptides form a random coil structure in water or PBS solution, while in a 50% H₂O/TFE, they take on a more orderly structure. This is best seen for both IL-4 derived peptides, which predominantly adopt a helical conformation. Therefore, CD analysis has provided valuable insights into the conformational changes of peptides under varying solvent conditions. The transition from disordered to helical conformations suggests a potential mechanism similar to that induced upon receptor binding. Induced fit involves the dynamic adjustment of both ligand and receptor conformations to optimize binding affinity, which could have significant implications for peptide-receptor interactions.

### Microscale Thermophoresis (MST) analysis of the interactions between IL-4 or IL-4-derived peptides and IL-4Rα

Interaction of the designed peptides with the protein was assessed using the microscale thermophoresis technique (MST). This method assesses the strength of the ligand-receptor interaction by determining the K_{D} value. The MST technique was used to investigate the interactions between IL-4 protein and two IL-4 derived peptides, SEQ ID NO: 1 (IL-4-X) or SEQ ID NO: 2 (IL-4-Y), with IL-4Rα. We were able to establish the equilibrium dissociation constants K_{D}, estimated in pseudo-titration mode for IL-4, calculated as 1.7 µM and for SEQ ID NO: 2 (IL-4-Y) peptide, calculated as 349 µM (Figure 2). Binding affinity between SEQ ID NO: 1 (IL-4-X) peptide and IL-4Rα using MST was not observed, indicating this peptide does not bind to the targeted receptor, however, MST measurements for short helical fragments can be unreliable and may underreport weak or transient interactions. In the case of the SEQ ID NO: 3 (SDF-1-X) and SEQ ID NO: 4 (SDF-1-Y) peptides, it was not possible to measure MST due to the fact that the CXCR4 receptor is a membrane protein. As of today, it remains very difficult to perform MST tests on transmembrane GPCR receptor systems.

Along with the structural analyses we studied the impact of the SEQ ID NO: 1 (IL-4-X), SEQ ID NO: 2 (IL-4-Y), SEQ ID NO: 3 (SDF-1-X), and SEQ ID NO: 4 (SDF-1-Y) at *in vitro* cultured cells. We aimed to test how these peptides, originating from proteins playing a crucial role in stem cell migration and myogenic differentiation, will influence cell proliferation migration, and differentiation. We chose to analyze model human cells, i.e., fibroblasts - hDFs, myoblasts - hSkMs, as well as two types of cells which can be used to support skeletal muscle regeneration, i.e., bone marrow stromal/medicinal signaling cells - hBMSCs and induced pluripotent stem cells - hiPSCs.

### Expression and activation of SDF-1 and IL-4 receptors

Expression of SDF-1 and IL-4 receptors was evaluated. SDF-1 acts via two receptors, i.e., CXCR4 and CXCR7, and IL-4 via IL4Rα heterodimer with either IL13Rα1 or γC. The level of mRNA in hDFs, hSkMs, hBMSCs, and hiPSCs was analyzed (Figure 5). The selected cell lines represent the endogenous or exogenous cell types that could be involved in skeletal muscle regeneration. The expression of *CXCR4, ACKR3* (encoding CXCR7), *IL-4Rα, IL-13Rα1,* and *IL2RG* (encoding γC) was detected in all analyzed cell lines (Figure 5A). Next, the presence of CXCR4, CXCR7, IL-4Rα, and IL-13Rα1 was noticed in all analyzed cell lines at protein level using immunocytochemistry, except for CXCR4 in hBMSCs (Figure 5B). Thus, we showed that hDFs, hSkMs, hBMSCs, and hiPSCs could respond to SDF-1 or IL-4 treatment. Then, we treated hDFs either with custom-designed SEQ ID NO: 3 (SDF-1-X), SEQ ID NO: 4 (SDF-1-Y), SEQ ID NO: 1 (IL-4-X), SEQ ID NO: 2 (IL-4-Y) peptides or cultured in medium lacking peptides (control), and analyzed if the signaling was activated in these cells. Previously, we showed that the level of active CDC42 increased in SDF-1 treated cells. Thus, in the present study we showed that active CDC42 level increased in SEQ ID NO: 3 (SDF-1-X) treated cells, as compared to control, however the difference was not significant (Figure 6A, B). To assess if IL-4 signaling is active in analyzed cells we estimated the level of the phosphorylation of STAT6 transcription factor and found that it increased, but not significantly, in treated cells (Figure 6C, D). Thus, we proved that custom-designed SDF-1 and IL-4 peptides are able to activate their signaling pathways in treated cells.

### Migration and proliferation of cells in response to custom-designed SDF-1 and IL-4 peptides

The migration of hBMSCs, hiPSCs, hDFs, and hiPSCs in response to IL-4 or SDF-1 peptide treatment was examined using scratch assay. As control we have chosen either cells cultured in medium lacking peptides or those cultured in the presence of SEQ ID NO: 5 (RGD) peptide, which do not interact with IL-4 and SDF-1 receptors. SEQ ID NO: 5 (RGD) binds to integrins localized at some cells and via this interaction can modify various processes. IL-4 and SDF-1 peptides did not cause any changes in the actin cytoskeleton that is involved in migration control (Figure 7A). The functional cell analysis showed that hDFs migration significantly increased after 24h in the response to SEQ ID NO: 3 (SDF-1-X), SEQ ID NO: 4 (SDF-1-Y), SEQ ID NO: 1 (IL-4-X), and SEQ ID NO: 2 (IL-4-Y) peptides, as compared to control and also SEQ ID NO: 5 (RGD) treated cells (Figure 5B, C). hBMSCs migration significantly increased in the presence of SEQ ID NO: 3 (SDF-1-X), as compared to control. Migration of hBMSCs in the presence of SEQ ID NO: 1 (IL-4-X) was however significantly lower as compared to cells cultured in the presence of SEQ ID NO: 3 (SDF-1-X) (Figure 7C). hSkMs migration was significantly increased by SEQ ID NO: 2 (IL-4-Y), and SEQ ID NO: 3 (SDF-1-X), compared to SEQ ID NO: 5 (RGD). hiPSCs migration was significantly decreased by SEQ ID NO: 4 (SDF-1-Y), as compared to SEQ ID NO: 5 (RGD), and SEQ ID NO: 3 (SDF-1-X) (Figure 7C). To assess proliferation rate cells were cultured for 3 days in the presence of tested peptides. No significant differences were noticed in case of peptide-treated hDFs and hBMSCs in comparison neither to control non-treated cells, nor to SEQ ID NO: 5 (RGD) (Figure 8). In the case of hSkMs, proliferation in the presence of SEQ ID NO: 3 (SDF-1-X) was significantly lower than in IL-4-Y-treated cells. We did not observe significant differences as compared to RGD. Lastly, for hiPSCs treated either with SEQ ID NO: 1 (IL-4-X) or SEQ ID NO: 3 (SDF-1-X) the proliferation was significantly lower than in SEQ ID NO: 5 (RGD)-treated cells (Figure 8). Thus, we showed that tested peptides improve cell migration and its impact on proliferation depends on the type of analyzed cells.

### Multilineage cell differentiation in response to custom-designed SDF-1 and IL-4 peptides

To follow the impact of custom-designed peptides on differentiation the hDFs were cultured in chondrogenesis, osteogenesis, or adipogenesis promoting medium and hSkMs were cultured in myogenesis inducing medium with or without analyzed peptides (Figure 9, 10). hDFs did not undergo neither chondrogenic nor osteogenic differentiation and peptides did not impact these processes (Figure 9A, B). As no differentiated areas were detected in cultures treated with analyzed peptides, no significant differences between the variants were spotted (data not shown). The changes in adipogenesis were noticed, however, the differentiation ratio, i.e., the proportion of area occupied by stained cells versus total area of cells, did not exceed 0.6%. hDFs adipogenic differentiation was very limited and decreased when treated with DM supplemented with either SEQ ID NO: 1 (IL-4-X) or SEQ ID NO: 2 (IL-4-Y) (Figure 10A, B). Finally, the hSkMs myogenic differentiation was analyzed (Figure 10C, D). The fusion index of hSkMs cultured in differentiating medium (DM) reached approximately 30%. In the presence of SEQ ID NO: 1 (IL-4-X) myoblast fusion index was the highest, however the observed differences were not statistically significant.

### Conclusions from provided data

Within the IL-4 family, SEQ ID NO: 2 (IL-4-Y) shows stronger binding characteristics than SEQ ID NO: 1 (IL-4-X) (MST detects SEQ ID NO: 2 (IL-4-Y) binding albeit weakly; SEQ ID NO: 1 (IL-4-X) undetected by MST). Safety/Specificity: peptides did not induce chondrogenesis or osteogenesis; IL-4 peptides decreased adipogenesis; no cytoskeletal disruption; cell-type-specific effects on migration/proliferation; SEQ ID NO: 1 (IL-4-X) improved myoblast fusion. The dataset supports single-family usage (IL-4-derived or SDF-1-derived) depending on the desired outcome (e.g., myoblast fusion vs migration).

### Example

### Peptide synthesis, purification, and ion exchange

Peptides were obtained by Solid Phase Peptide Synthesis SPPS using Liberty Blue automated microwave synthesizer (CEM Corporation). TentaGel HL RAM resin (capacity 0.35 mmol/g) was used as a solid support along with 0.5 M DIC solution in DMF, 1 M solution of Oxyma Pure as reagents. For peptide cleavage, TFA with phenol, water, and TIPSI was used. The peptides were purified by preparative RP-HPLC on the Jupiter^{®} Proteo C12 column, 4 µm, 90 Å, 250 mm x 21.1 mm (Phenomenex) and analyzed using analytical RP-HPLC (Kinetex C8, 2.6 µm, 100 Å, 100 × 2.1 mm, Phenomenex) and mass spectrometry (LC-MS ESI-TOF, Shimadzu) (Table 2). The purified peptides, obtained as trifluoroacetate salts, were subjected to the counterion exchange to acetate.

### Secondary-structure analysis (CD)

For CD measurements, the stock solutions of pure peptides SEQ ID NO: 1 (IL-4-X), IL-4-Y, SEQ ID NO: 3 (SDF-1-X) and SEQ ID NO: 4 (SDF-1-Y) were diluted to 0.2 mg/ml. The spectra were obtained at room temperature on a Jasco J-815 spectropolarimeter(JASCO) equipped with 1-5 mm quartz cells. The results were plotted as the mean residue ellipticity [Θ] [deg×cm²×dmol⁻¹]. The spectra for peptides in water and trifluoroethanol (TFE) were recorded over wavelength range of 185-260 nm and in PBS over the wavelength range 195-260 nm.

### Evaluation of interactions between IL-4 or IL-4 derived peptides and IL4Rα using microscale thermophoresis (MST)

His-tagged human IL-4Rα protein (cat. No. ILR-H5221, Acro Biosystems, USA) was adjusted to concentration of 200 nM and labeled using a Monolith His-Tag Labeling Kit RED-tris-NTA 2nd Generation (NanoTemper Technologies) according to the manufacturer's instructions. The IL-4 protein (cat. No. 11846-HNAE, Sino Biological) and SEQ ID NO: 1 (IL-4-X) and SEQ ID NO: 2 (IL-4-Y) peptides were suspended in PBS-T buffer (PBS buffer 0.05% (v/v) TWEEN 20; pH 7.4), at a final concentration of 8.34 µM, 0.5 µM and 0.5 mM, respectively. To assess binding interactions between the IL-4Rα receptor, IL-4 protein and peptides, MST measurements were performed using a Monolith X instrument (NanoTemper Technologies). Samples were prepared according to the manufacturer's instructions and loaded into Premium Capillaries (NanoTemper Technologies). Medium MST power was applied at 25°C. Dose response curves in a function of 670 nm/650 nm ratio and dissociation constant (K_{D}) were calculated using MO. At least three independent measurements were performed for each sample.

### Cell lines and cell culture

Commercially available cell lines were used in the experiments: human Skeletal Myoblasts (hSkMs, A11440, Thermo Fisher), human Dermal Fibroblasts (hDFs, 106-05A, Sigma Aldrich), and bone marrow mesenchymal stem cells (hBMSCs; PT-2501, Lonza). In addition, IIMCB1002-A human induced pluripotent stem cells (hiPSCs) were used [43]. All cells were used from passages 1-6. hSkMs were maintained in hSkM-growth medium (hSkM-GM) consisting of 84% Dulbecco's Modified Eagle's Medium, low glucose (21885-025, Gibco), 15% heat inactivated fetal bovine serum (FBSin, 10500-064, Gibco), and 1% penicillin/streptomycin (15070-063, Gibco). hDFs were cultured in hDF-growth medium (hDF-GM) consisting of 89% Dulbecco's Modified Eagle's Medium, low glucose (21885-025, Gibco), 10% FBSin (10500-064, Gibco) and 1% penicillin/streptomycin (15070- 063, Gibco). hBMSCs were cultured in hBMSC-growth medium (hBMSC-GM, Dulbecco's Modified Eagle's Medium, high glucose (31966-047, Gibco), 10% FBSin (Thermo Fisher) and 1% penicillin/streptomycin (15070-063, Gibco). hiPSCs were cultured in Essential 8^{™} Medium (A28585-01, Thermo Fisher) supplemented with 1% gentamicin (G1272-100ML, Sigma Aldrich) on dishes coated with Matrigel^{®} Growth Factor Reduced Basement Membrane Matrix (734-0268, VWR) diluted 1:100 in cold DMEM/F-12 (10565-018, Gibco). To assess the influence of various peptides on cells the media were supplemented with one of the peptides, i.e., either with SEQ ID NO: 1 (IL-4-X), SEQ ID NO: 2 (IL-4-Y), SEQ ID NO: 3 (SDF-1-X), SEQ ID NO: 4 (SDF-1-Y), or SEQ ID NO: 5 (RGD), at concentration of 250 ng/mL. The media supplemented with peptides are marked here with the suffix "-S". Cell media were changed every 2-3 days.

### Proliferation Assay

To assess the proliferation hSkMs, hDFs, hBMSCs, or hiPSCs were seeded in 24-well plates, at the density of 5000 cells/cm². The cells were maintained in dedicated culture media, being either control ones, i.e., without peptides, or hSkM-GM-S, hDF-GM-S, hBMSC-GM-S, Essential 8^{™} Medium-S, respectively. After 3 days in culture, cells were trypsinized and counted in the Malassez chamber. The results were shown as the mean number of cells ± standard deviation. Three independent experiments were performed.

### Migration Assay

Migration of hSkMs, hDFs, hBMSCs, or hiPSCs was assessed using scratch wound healing assay, based on previously published protocol. Briefly, the cells were seeded in 35 mm dishes and cultured in media supplemented with peptides until reaching 90-100% confluence. Control cells were cultured in media lacking peptides. The cells were next scratched using a plastic tip to create 3 "wounds" in the middle of the wells. The area was observed and photographed immediately after performing scratch (time "0") and after 24 hours, when cells were fixed with 3% paraformaldehyde (3% PFA) in PBS and stained with Giemsa (1.09204.0500, Sigma Aldrich) - May-Grünwald (1.01424.0500, Sigma Aldrich). Then the cells were photographed using Nikon Eclipse TE200 microscope and Nikon Digital Sight DS-U2 camera. The captured photos were analyzed using Image J (NIS-Elements F 2.30). Three independent experiments were performed.

### Human Dermal Fibroblast Differentiation

Differentiation of hDFs was induced by using special media: for chondrogenesis - ChondroMAX (SCM123, Sigma Aldrich), for adipogenesis - AdipoMAX (SCM122, Sigma Aldrich), and for osteogenesis - OsteoMAX (SCM121, Sigma Aldrich). Initially, hDFs were seeded in 24-well plates and maintained either in control hDF-GM or in hDF-GM-S until reaching confluence. Next, the cells were exposed to abovementioned special media lacking (control) or supplemented with tested peptides. hDFs were cultured for either 19, 12 or 7 days to assess chondrogenic, adipogenic, or osteogenic differentiation, respectively. Next, cells were rinsed with PBS and fixed with 3% PFA in PBS and stained with Alcian Blue (TMS-010, Sigma Aldrich) to visualize acidic polysaccharides in cartilage, Oil Red O (O1391-500ML, Sigma Aldrich) to stain lipid droplets, or Alizarin Red S (TMS-008-C, Sigma Aldrich) to identify calcium residues, according to manufacturer's protocols. Specimens were next photographed using Nikon Eclipse TE200 microscope and Nikon Digital Sight DS-U2 camera. The captured photos were analyzed using Image J (NIS-Elements F 2.30). Differentiation ratio was calculated as the area of differentiated cells divided by the total area of cells in the photo. Data quantification was performed using GraphPad Prism 9 Software. Three independent experiments were performed.

### Human Skeletal Myoblast Differentiation and Fusion Index

hSkMs were seeded into 24-well plates at the density of 30 000 cells/cm². The cells were cultured in either control hSkM-GM or hSkM-GM-S until reaching confluence. Then the medium was changed to differentiating medium (hSkM-DM, 97% Dulbecco's Modified Eagle's Medium, low glucose (Thermo Fisher), 2% horse serum (HS, Thermo Fisher), 1% penicillin/streptomycin (Thermo Fisher)], either control, i.e. lacking peptides, or supplemented with tested peptides. Cells were kept in culture for 3 days until myotubes were formed. Then, the cells were rinsed with PBS and fixed with 3% PFA in PBS. After another PBS washing, the cells were stained with Giemsa-May Grünwald, according to previously described protocol. Fixed and stained cells were next photographed using Nikon Eclipse TE200 microscope and Nikon Digital Sight DS-U2 camera. The captured photos were analyzed using Image J (NIS-Elements F 2.30). Fusion index was calculated as the number of nuclei inside myotubes (containing at least three nuclei) divided by the total number of nuclei in the photo. Three independent experiments were performed.

### RT-qPCR

Total RNA was isolated from cell pellets using High Pure RNA Isolation Kit (11.828.665.001, Roche), according to the manufacturer's protocol. Next, the determination of RNA concentration and contamination using NanoDrop (Thermo Scientific) was performed. Then, reverse transcription was executed using RevertAid First Strand cDNA Synthesis Kit (K1622, Thermo Fisher), according to manufacturer instructions using 0.5 µg RNA for each reaction. Quantitative Polymerase Chain Reaction (qPCR) was performed using TaqMan Gene Expression Master Mix (4369016, Thermo Fisher) and TaqMan probes, according to previously published protocol. Actin B was used as a reference gene. Results analysis was performed with LightCycler^{®} 96 Software 1.1 (Roche) and Graph Pad Prism 9. The data was shown as Delta CT values. The following TaqMan probes were used: IL13Rα1 (Hs00609812_m1, Thermo Fisher), IL4Rα (Hs00965056_m1, Thermo Fisher), IL2RG (encoding γC; Hs00415671_m1, Thermo Fisher), CXCR4 (Hs00976734_m1, Thermo Fisher), ACKR3 (encoding CXCR7; Hs00664172_s1, Thermo Fisher), ACTB (Hs01060665_g1, Thermo Fisher). Three independent experiments were performed.

### Western Blot Analysis

hDFs were cultured in 100 mm cell culture dishes in hDF-GM until confluence and then in hDF-GM-S for 24 hours. Next, the cells were rinsed with cold PBS and collected, using cell scraper and Lysis/Binding/Wash Buffer (1862301, Thermo Scientific) supplemented with protease inhibitors (11836153001, Roche) and PhosSTOP (4906845001, Sigma Aldrich). Collected cells were vortexed, chilled on ice for 5 minutes, and then centrifuged (14000 RPM, at 4°C, 15 minutes). Finally, the supernatants were collected. The protein concentration was measured using the Pierce BCA protein Assay Kit (23225, Thermo Scientific). Ten micrograms of protein samples were used in the case of STAT6-P and 50 µg in case of CDC42 immunodetection. The samples were boiled in 2X Laemmli Sample Buffer (161-0737, Bio-Rad) for 5 minutes, then separated in sodium dodecyl sulfate polyacrylamide gel electrophoresis and transferred to polyvinylidene difluoride membrane (162-0177, Bio-Rad), according to previously described protocol. Membranes were then washed twice in 0.05% Tween-Tris Buffered Saline (TTBS) and blocked in 5% non-fatty dry milk in TTBS in room temperature with gentle rocking for 1 hour. Next, the membranes were incubated with primary antibodies (1:1000 in 2.5% non-fatty dry milk in TTBS) on laboratory cradle, at 4°C, overnight. The following day, membranes were washed 4 times in TTBS and incubated with a secondary antibody conjugated with horseradish peroxidase (1:10 000 in 5% non-fatty dry milk in TTBS) at room temperature, for 1 hour. Then membranes were washed 4 times in TTBS and additional 2 times in TBS. In order to visualize protein bands, membranes were incubated with Super Signal^{™} West Dura Extended Duration Substrate (34076, Thermo Scientific), for 10 minutes, at room temperature, protected from light. Next, the membranes were exposed to chemiluminescence positive film (28906837, Amersham Hyperfilm ECL). The following primary antibodies were used: murine monoclonal anti-CDC42 Antibody (1862345, Thermo Scientific), rabbit monoclonal anti-STAT6-P Antibody (56554, Cell Signaling), rabbit monoclonal anti-β-tubulin Antibody (2146, Cell Signaling). The following secondary antibodies were used: peroxidase conjugate rabbit anti mouse (A9044, Sigma Aldrich), peroxidase conjugate goat anti rabbit (A9169, Sigma Aldrich). Protein levels were quantified using Gel Doc XR+ (Bio-Rad, Hercules, CA, USA) and its optical density was compared with tubulin bands. The statistical analysis was performed in GraphPad Prism 9. Three independent experiments were performed.

### Immunocytochemistry

hSkMs, hDFs, hBMSCs, and hiPSCs were cultured on coverslips coated with 1% gelatin until reaching 80-90% confluence. Then, cells were fixed with 3% PFA (Paraformaldehyde, P6148-500G, Sigma Aldrich) and cell membranes were permeabilized by 1 minute incubation with 0.05% Triton X-100 (T8787, Sigma Aldrich) in PBS. Next, in order to block free aldehyde groups, the cells were incubated with 0.25% glycine in PBS, following 1 hour incubation with 3% Bovine Serum Albumin (BSA) in PBS to block nonspecific antigen binding sites. Then, overnight incubation with primary antibody, at 4°C, was performed, followed by three washes with PBS. Then, 2 hour incubation with secondary antibody in PBS was performed and cells were washed three times with PBS. Next, cells were mounted to slides (S3023, Dako). The following primary antibodies were used: rabbit polyclonal anti-IL4Rα (NBP1-00884, Novus Biologicals), rabbit polyclonal anti-IL13Rα1 (NBP1-61690, Novus Biologicals), rabbit monoclonal anti-CXCR4 (ab181020, Abcam), rabbit monoclonal anti-CXCR4 (ab208128, Abcam), rabbit polyclonal anti-GPCR RDC1/CXCR-7 (ab117836, Abcam) or rabbit polyclonal anti-GPCR RDC1/CXCR-7 (ab137485, Abcam) followed by donkey anti-rabbit (A-21207, Thermo Fisher) secondary antibody. Cell nuclei were stained for 5 minutes with Hoechst 33342 (B-2261, Sigma Aldrich) diluted 1:1000 in PBS. To visualize actin cytoskeleton Phalloidin-Tetramethyl-rhodamine B isothiocyanate (P1951, Sigma-Aldrich), diluted 1:100 in PBS was used. Three independent experiments were performed.

### Industrial applicability

The peptides and compositions of the invention are applicable in: (a) Regenerative medicine and tissue engineering, as additives to culture media or coatings for muscle-derived cells and constructs. (b) Pharmaceutical and biotechnology manufacturing, where short, defined peptides provide safer alternatives to recombinant cytokines. (c) Medical devices and bioactive scaffolds enabling localized release of pro-regenerative signals in injured skeletal muscle. (d) In-vitro testing and high-throughput assays for drug discovery on myogenic or stromal cell migration and differentiation. (e) The sequences can be synthesized by standard solid-phase chemistry, purified by HPLC, and stored in lyophilized form for distribution and long-term use. (f)

### Abbreviations

SCs: Satellite cells, a muscle-resident stem cells
MPCs: Myogenic precursor cell derived from SCs or PSCs
hSkMs: Human skeletal myoblasts
hDFs: Human dermal fibroblasts
hBMSCs: Human bone-marrow mesenchymal stromal cells
hiPSCs: Human induced pluripotent stem cells
SEQ ID NO: 1 (IL-4-X) / SEQ ID NO: 2 (L-4-Y): IL-4(5-15) and IL-4(78-92) derived peptides, respectively
SEQ ID NO: 3 (SDF-1-X) / SEQ ID NO: 4 (SDF-1-Y): SDF-1(1-8) and SDF-1(39-49) derived peptides, respectively
MMP: Matrix metalloproteinase
SEQ ID NO: 5 (RGD): Arg-Gly-Asp adhesion tripeptide (control)

### References:

[1] B. Mierzejewski et al., Human and mouse skeletal muscle stem and progenitor cells in health and disease, Semin Cell Dev Biol 104 (2020) 93-104.
[2] C. Rodriguez et al., Cellular interactions and microenvironment dynamics in skeletal muscle regeneration and disease, Front Cell Dev Biol 12 (2024) 1385399.
[3] K. Sahinyan et al., Decline of regenerative potential of old muscle stem cells: contribution to muscle aging, FEBS J 290(5) (2023) 1267-1289.
[4] G. Careccia et al., Regulation of Satellite Cells Functions during Skeletal Muscle Regeneration: A Critical Step in Physiological and Pathological Conditions, Int J Mol Sci 25(1) (2023).
[5] M. Shayan, N.F. Huang, Pre-Clinical Cell Therapeutic Approaches for Repair of Volumetric Muscle Loss, Bioengineering (Basel) 7(3) (2020).
[6] Y. Fan et al., Rapid death of injected myoblasts in myoblast transfer therapy, Muscle Nerve 19(7) (1996) 853-60.
[7] M. Bouchentouf et al., Induction of Anoikis following myoblast transplantation into SCID mouse muscles requires the Bit1 and FADD pathways, Am J Transplant 7(6) (2007) 1491-505.
[8] D. Skuk, J.P. Tremblay, Cell Therapy in Myology: Dynamics of Muscle Precursor Cell Death after Intramuscular Administration in Non-human Primates, Mol Ther Methods Clin Dev 5 (2017) 232-240.
[9] C. Gerard, M.A. Forest, G. Beauregard, D. Skuk, J.P. Tremblay, Fibrin gel improves the survival of transplanted myoblasts, Cell Transplant (2011).
[9] C. Gerard, M. A. Forest, G. Beauregard, D. Skuk, and J. P. Tremblay, "Fibrin Gel Improves the Survival of Transplanted Myoblasts," Cell Transplantation 21 (2011): 127-137.
[10] C.A. Cezar, D.J. Mooney, Biomaterial-based delivery for skeletal muscle repair, Adv Drug Deliv Rev 84 (2015) 188-97.
[11] M. Quattrocelli et al.mpaolesi, Notch signaling regulates myogenic regenerative capacity of murine and human mesoangioblasts, Cell Death Dis 5 (2014) e1448.
[12] A. Dellavalle et al., Pericytes of human skeletal muscle are myogenic precursors distinct from satellite cells, Nat Cell Biol 9(3) (2007) 255-67.
[13] K.J. Mitchell et al., Identification and characterization of a non-satellite cell muscle resident progenitor during postnatal development, Nat Cell Biol 12(3) (2010) 257-66.
[14] K.K. Tanaka et al., Syndecan-4-expressing muscle progenitor cells in the SP engraft as satellite cells during muscle regeneration, Cell Stem Cell 4(3) (2009) 217-25.
[15] M.A. LaBarge, H.M. Blau, Biological progression from adult bone marrow to mononucleate muscle stem cell to multinucleate muscle fiber in response to injury, Cell 111(4) (2002) 589-601.
[16] I. Grabowska et al., Adipose Tissue-Derived Stromal Cells in Matrigel Impacts the Regeneration of Severely Damaged Skeletal Muscles, Int J Mol Sci 20(13) (2019).
[17] K. Archacka et al., Pluripotent and Mesenchymal Stem Cells - Challenging Sources for Derivation of Myoblast, in: Z. Brzozka, E. Jastrzebska (Eds.), Cardiac Cell Culture Technologies, Springer International Publishing2018, pp. 109-154.
[18] E. Shoji et al., Directed Myogenic Differentiation of Human Induced Pluripotent Stem Cells, Methods Mol Biol 1353 (2016) 89-99.
[19] E. van der Wal et al., Large-Scale Expansion of Human iPSC-Derived Skeletal Muscle Cells for Disease Modeling and Cell-Based Therapeutic Strategies, Stem cell reports 10(6) (2018) 1975-1990.
[20] J.F. Lafreniere, P. Mills, M. Bouchentouf, J.P. Tremblay, Interleukin-4 improves the migration of human myogenic precursor cells in vitro and in vivo, Exp Cell Res 312(7) (2006) 1127-41.
[21] V. Horsley et al., IL-4 acts as a myoblast recruitment factor during mammalian muscle growth, Cell 113(4) (2003) 483-94.
[22] K. Kowalski et al., Stem cells migration during skeletal muscle regeneration - the role of Sdf-1/Cxcr4 and Sdf- 1/Cxcr7 axis, Cell Adh Migr 11(4) (2017) 384-398.
[23] E. Brzoska et al., Sdf-1 (CXCL12) improves skeletal muscle regeneration via the mobilisation of Cxcr4 and CD34 expressing cells, Biol Cell 104(12) (2012) 722-37.
[24] B. Swierczek-Lasek et al., Interleukin 4 Moderately Affects Competence of Pluripotent Stem Cells for Myogenic Conversion, Int J Mol Sci 20(16) (2019).
[25] K. Archacka et al., Beneficial Effect of IL-4 and SDF-1 on Myogenic Potential of Mouse and Human Adipose Tissue-Derived Stromal Cells, Cells 9(6) (2020).
[26] P. Kasprzycka et al., The factors present in regenerating muscles impact bone marrow-derived mesenchymal stromal/stem cell fusion with myoblasts, Stem cell research & therapy 10(1) (2019) 343.
[27] E. Brzoska, I. Grabowska, G. Hoser, W. Streminska, D. Wasilewska, E.K. Machaj, Z. Pojda, J. Moraczewski, J. Kawiak, Participation of stem cells from human cord blood in skeletal muscle regeneration of SCID mice, Exp Hematol 34(9) (2006) 1262-70.
[28] K. Kowalski et al., Stromal derived factor-1 and granulocyte-colony stimulating factor treatment improves regeneration of Pax7-/- mice skeletal muscles, J Cachexia Sarcopenia Muscle 7(4) (2016) 483-96.
[29] E. Brzoska et al., Sdf-1 (CXCL12) induces CD9 expression in stem cells engaged in muscle regeneration, Stem cell research & therapy 6 (2015) 46.
[30] Q. Li et al., Stromal cell-derived factor-1 promotes human adipose tissue- derived stem cell survival and chronic wound healing, Exp Ther Med 12(1) (2016) 45-50.
[31] Z. Julier et al., Promoting tissue regeneration by modulating the immune system, Acta Biomater 53 (2017) 13-28.
[32] S.A. Aaronson, Growth-Factors and Cancer, Science 254(5035) (1991) 1146-1153.
[33] M. Deptula et al., Development of a Peptide Derived from Platelet-Derived Growth Factor (PDGF-BB) into a Potential Drug Candidate for the Treatment of Wounds, Adv Wound Care (New Rochelle) 9(12) (2020) 657-675.
[34] E. Kim, F. Wu, X. Wu, and H. J. Choo, "Generation of Craniofacial Myogenic Progenitor Cells From Human Induced Pluripotent Stem Cells for Skeletal Muscle Tissue Regeneration," Biomaterials 248 (2020): 119995.
[35] C. Ligorio, A. Mata, Synthetic extracellular matrices with function-encoding peptides, Nat Rev Bioeng (2023) 1-19.
[36] M. Zimowska et al., IL-4 and SDF- 1 Increase Adipose Tissue-Derived Stromal Cell Ability to Improve Rat Skeletal Muscle Regeneration, Int J Mol Sci 21(9) (2020).

## Claims

1. A synthetic peptide selected from:
a) LITLQEIIKTLN (SEQ ID NO: 1, IL-4-X),
b) LQLIRFLKRLDRNLWG (SEQ ID NO: 2, IL-4-Y),
c) LKPVSLSYR (SEQ ID NO: 3, SDF-1-X),
d) LVARLKNNNRQV (SEQ ID NO: 4, SDF-1-Y),
wherein the said peptide comprises an N-terminal Leu residue enabling MMP-sensitive conjugation.

2. A peptide-based composition for modulating cellular processes involved in skeletal muscle regeneration, wherein the composition comprises at least one synthetic peptide selected from SEQ ID NO: 1-4, wherein each peptide is covalently tethered to a biomaterial scaffold through an MMP-cleavable linker selected from VLG↓L, PLG↓L, PAG↓L, PLN↓L, PAN↓L or VAL↓L.

3. The composition according to claim 2, wherein the synthetic peptide is SEQ ID NO: 1 or SEQ ID NO: 2, and wherein said peptide binds to and activates the IL-4 receptor α to enhance fusion of skeletal myoblasts and decrease adipogenic differentiation of fibroblasts.

4. The composition according to claim 2, wherein the synthetic peptide is SEQ ID NO: 3 or SEQ ID NO: 4, and wherein said peptide binds to and activates CXCR4 receptors to enhance migration of fibroblasts, skeletal muscle precursor cells, or mesenchymal stromal cells.

5. The composition according to claim 2, wherein the biomaterial scaffold is chosen from the list: PEG-based hydrogels, chitosan sponges, and polymeric, synthetic hydrogels.

6. The composition of any preceding claim, wherein the synthetic peptide is present at a concentration from 50 ng/mL to 1000 ng/mL, preferably about 250 ng/mL

7. The synthetic peptide according to claim 1 or the peptide-based composition according to any of claim 2 to 6 for use as a medicament.

8. The synthetic peptide according to claim 1 or the peptide-based composition according to any of claim 2 to 6 for use in enhancing skeletal muscle repair and promoting regeneration of skeletal muscle by modulating human dermal fibroblasts, skeletal myoblasts, bone-marrow mesenchymal stromal cells induced pluripotent stem cells.

9. A use of the synthetic peptide according to claim 1 or the peptide-based composition according to any of claim 2 to 6 for promoting regeneration of injured skeletal muscle in a mammal.

10. The use according to claim 8, wherein said composition is administered together with induced pluripotent stem cells, or myogenic cells (myoblasts) to further enhance skeletal muscle repair.
